(19) Europäisches Patentamt
European Patent Office
Office européen des brevets

(11) **EP 2 946 324 B1**

## (12) EUROPEAN PATENT SPECIFICATION

(45) Date of publication and mention
of the grant of the patent:
**10.11.2021 Bulletin 2021/45**

(21) Application number: **14740511.2**

(22) Date of filing: **14.01.2014**

(51) Int Cl.:
*G06Q 10/10* (2012.01)    *G16H 50/70* (2018.01)

(86) International application number:
**PCT/IL2014/050048**

(87) International publication number:
**WO 2014/111933 (24.07.2014 Gazette 2014/30)**

(54) **MEDICAL DATABASE AND SYSTEM**

MEDIZINISCHE DATENBANK UND SYSTEM DAMIT

BASE DE DONNÉES MÉDICALES ET SYSTÈME CORRESPONDANT

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.01.2013 US 201361753378 P**

(43) Date of publication of application:
**25.11.2015 Bulletin 2015/48**

(73) Proprietor: **MedAware Ltd.**
**4366238 RaAnana (IL)**

(72) Inventor: **BEKER, Tuvia**
**4728418 Ramat-HaSharon (IL)**

(74) Representative: **Michalski Hüttermann & Partner
Patentanwälte mbB
Speditionstraße 21
40221 Düsseldorf (DE)**

(56) References cited:
**US-A1- 2003 204 415    US-A1- 2007 219 824
US-A1- 2009 216 555    US-A1- 2011 106 733
US-B1- 8 046 242    US-B2- 7 197 492
US-B2- 7 418 299    US-B2- 7 643 969**

**Description**

FIELD AND BACKGROUND OF THE INVENTION

The present invention relates to a medical database representing the medical file of a subject as a multi-element data module and to systems and methods of using same to identify medically-relevant information (e.g. potential medication incompatibility) not present in the medical file of the subject.

Prescription errors account for 70% of adverse medication errors [1], with in-hospital prescription errors found in 8.9% of prescriptions [2] with similar numbers found in an outpatient setting [3]. According to IMS Vector One pharmacy data, prescribing errors occur in 7.6% of outpatient prescriptions, and 50% of the prescribing errors are deemed dangerous. The annual cost of medication errors in the United States is estimated at $21B [4]. According to recent studies "... the true number of premature deaths associated with preventable harm to patients was estimated at more than 400,000 per year. Serious harm seems to be 10- to 20-fold more common than lethal harm" [5]. It is now accepted that prescription errors depend on failures of individuals, but are facilitated by failures in medical systems [1].

In recent years, emerging Electronic Medical Records (EMR) and Computerized Physician Order Entry (CPOE) technologies have been entering the clinical arena. These technologies significantly lower the incidence of prescription errors, by identifying dosage errors, incompatible drug interactions and allergies [1, 6-8]. However, only 53% of fatal medication orders are identified by implemented commercial computerized physician order entry systems [9]. Moreover, the growing dependence on EMR systems has led to an increase in human errors resulting in prescription mix-ups in which a drug is prescribed to the wrong patient [10].

There is thus a need for a system which can identify medically-relevant information, such as drug prescription compatibility, in a medical file of a subject and thus prevent potential prescription errors in cases where such potential errors would otherwise go undetected by present day systems.

US 2007/219824A1 discloses a method for identifying aberrations in a patient's treatment pattern for a medical condition, aberrations in treatment patterns representing treatment of a number of patients for the same medical condition by a healthcare provider, or aberrations in patterns based upon other claim data groupings as desired by the user.

US 2009/216555 A1 discloses a system, method and computer program product for automatically detecting and tracking adverse events, as well as automatically generating one or more performance metrics associated with the detected adverse events, and enabling the performance of an in depth root cause analysis of the detected adverse events.

SUMMARY OF THE INVENTION

The present invention relates to a data system as defined in the appended set of claims.

Disclosed is also a medical data system comprising a data unit for storing modules representing medical records of subjects, each module including a plurality of module elements each representing a medically-relevant parameter of a subject, wherein each element is assigned a specific identifier in the module and a numerical value corresponding to the medically-relevant parameter.

According to examples described below, the numerical value can be a Boolean, discrete or continuous numerical value.

According to still further examples, the discrete or continuous numerical value is normalized.

According to still further examples, the medically-relevant parameter is selected from the groups consisting of a demographic parameter, a physiological parameter, a drug prescription-related parameter, a disease related parameter, and a treatment related parameter.

According to still examples, the medical data system further comprises an inference engine for comparing, based on the identifiers, values of at least a portion of the elements of the module of the subject to a plurality of modules of diagnosed/qualified subjects or to at least one model constructed from statistical characteristics of historical data of diagnosed/qualified subjects to thereby identify medically-relevant information not present in a medical file of the subject.

According to still further examples, the medically-relevant information is a probable drug prescription error.

According to still further examples, the probable prescription error is based on frequency of prescription of the drug in diagnosed subjects having module elements with values within a predetermined distance from values of respective module elements of the subject.

According to still further examples, the predetermined distance is determined by embedding the modules in a vector space through a smooth mapping function and then measuring the distance between the mapped points in that space using a metric induced by a properly defined norm in the vector space.

According to still further examples, the probable prescription error is based on binary classification based on the at least one model.

According to still further examples, the probable prescription error is based on continuous regression against the at least one model.

According to still further examples, the medical data system further comprises a user interface for displaying the medically-relevant information to a physician.

According to still further examples, the medical data system further comprises a learning engine for assimilating a response of the physician to the information into the at least one model.

According to still further examples, the module is arranged as a finite dimension vector having a preset length.

According to still further examples, the vector represents a time-related pattern of demographic data, prescriptions, diagnoses, hospitalizations, lab test results and/or medical procedures.

According to another aspect of the disclosure, there is provided a method of representing medical data of a subject comprising processing medical records of the subject to convert each medically-relevant parameter of a subject into module elements and assembling a plurality of module elements into a module, wherein each element is assigned a specific identifier in the module and a numerical value corresponding to the medically-relevant parameter.

According to yet another aspect of the disclosure, there is provided a method of identifying medically-relevant information not present in a medical file of a subject comprising: (a) providing a module including a plurality of module elements each representing a medically-relevant parameter of the subject, wherein each element is assigned a specific identifier in the module and a numerical value corresponding to the medically-relevant parameter; and (b) comparing, based on the identifiers, values of at least a portion of the elements of the module of the subject to a plurality of modules of diagnosed subjects or to at least one model constructed from statistical characteristics of historical data of diagnosed subjects to thereby identify medically-relevant information not present in a medical file of the subject.

According to still further examples, the medically-relevant information is a probable drug prescription error.

According to still further examples, the probable prescription error is based on frequency of prescription of the drug in diagnosed subjects having module elements with values within a predetermined distance from values of respective module elements of the subject.

According to still further examples, the predetermined distance is determined by embedding the modules in a vector space through a smooth mapping function and then measuring the distance between the mapped points in that space using a metric induced by a properly defined norm in the vector space.

According to still further examples, the probable prescription error is based on binary classification based on the at least one model.

According to still further examples, the probable prescription error is based on continuous regression against the at least one model.

According to still further examples, the method further comprises displaying the medically-relevant information to a user.

According to still further examples, the method further comprises assimilating a response of the user to the information into the at least one model.

According to still further examples, the module is arranged as a finite dimension vector having a preset length.

According to still further examples, the vector represents a time-related pattern of demographic data, prescriptions, diagnoses, hospitalizations, lab test results and/or medical procedures.

The present invention successfully addresses the shortcomings of the presently known configurations by providing a medical database and system which can be used to infer medically relevant information such as drug prescription errors from a patient's medical file.

Implementation of the method and system of the present invention involves performing or completing selected tasks or steps manually, automatically, or a combination thereof. Moreover, according to actual instrumentation and equipment of preferred embodiments of the method and system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the method and system of the invention could be described as being performed by a data processor, such as a computing platform for executing a plurality of instructions.

BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:

FIG. 1 is a block diagram illustrating the present system.

FIG. 2 is a flowchart illustrating construction of a medical record data module (vector) according to the teachings of the present invention.

FIG. 3 is a graph showing distribution of patients on or off statin treatment.

DESCRIPTION OF THE PREFERRED EMBODIMENTS

The present invention is of a medical database and of systems and methods for generating and using the database to derive medically-relevant information not present in a medical file of a subject. Specifically, the present invention can be used to provide alerts with respect to drug prescription errors in cases where such potential errors would otherwise go undetected by present day EMR systems.

The principles and operation of the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

Electronic medical record (EMR) systems organize a patients medical record as a knowledge-base file which correlates patient characteristics (medical profile) with patient prescription logs and thus such systems enable identification of possible drug prescriptions errors. For example, a prescription of a drug which cannot be used during pregnancy can be flagged as an error in cases where a patient's file indicates pregnancy while a prescription for a drug which has known drug interactions would be flagged as a possible error in cases where the medical record of a patient indicates such possible interactions.

In such simple cases an EMR system provides sufficient information to identify potentially harmful prescriptions. However, since such systems only analyze a specific patient file, they are limited to the information contained therein and thus cannot derive medically-relevant information which can be extrapolated from the medical file of a patient. Thus, while an expert physician can derive further (albeit limited) information from a medical file of a patient based on experience and prior knowledge relating to other patients, an EMR system is incapable of such functionality.

While reducing the present invention to practice, the present inventor devised a medical record system which represents a medical file of a patient in a manner which enables modeling and clustering of medical record data from a plurality of patients.

The present system utilizes the modeled/clustered medical data to perform real-time evaluation of specific medical parameters (e.g. prescribed drugs) against a patient's individual profile (represented by multi-element data module, e.g. vector) in order to identify potential problems such as prescription errors. Such problems can then be communicated (e.g. as an alert of potentially harmful prescription errors) to a technician or physician.

Since the present system enables extraction of medically-relevant information not present in a patient's file, it can identify drug prescription errors as well as other parameters that would not be available from EMR systems.

Thus, according to one aspect of the present invention there is provided a medical data system for storing medical records of subjects. The medical data system of the present invention enables modeling and clustering of medical data, as well as analyzing subject-specific medical data with respect to modeled/clustered data.

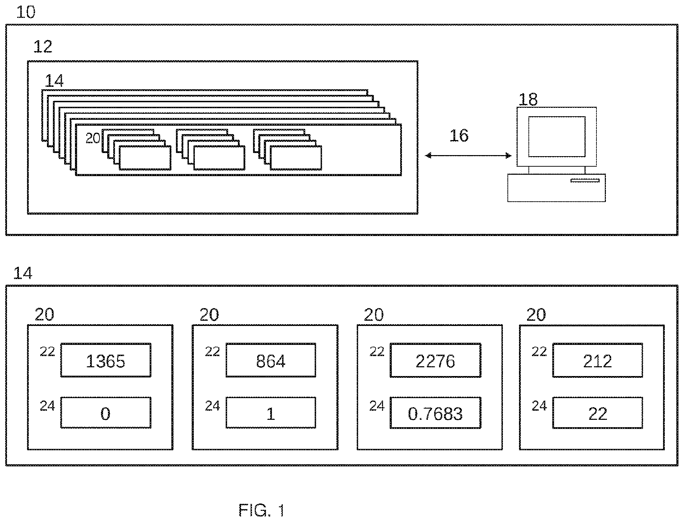

Figure 1 illustrates the medical database system of the present invention which is referred to herein as system 10.

System 10 includes a data unit 12 for storing modules 14 representing medical records of subjects. Modules 14 are stored as records in a database such as a standard relational database, as rows in a delimiter-separated text file, or in any such other data storage format. Data unit 12 is stored on a user accessible storage medium (magnetic/optical drive) of a computer platform such as a desktop, laptop, work station, server and the like. The computing platform can be accessed locally or through a communication network 16 through any computing devices 18 including stationary (e.g. desktop computers) and mobile (e.g. smartphones, tablets etc) devices.

Each module 14 includes a plurality of module elements 20 each representing a medically-relevant parameter of a subject. Each element 20 is assigned a specific identifier 22 in the module and a numerical value 24 corresponding to the medically-relevant parameter. The numerical value can be a Boolean, discrete or continuous numerical value.

Examples of medically relevant parameters includes, but are not limited to, time-related parameters such as time period of hospitalization, time period since last blood test, (for example, results of blood levels of a drug who should be closely monitored, such as INR monitoring for Warfarin, will more relevant if taken within the previous week and irrelevant if taken a year ago), prescription related parameters such as drugs prescribed (for example, if a drug and it's antidote are prescribed simultaneously, such as Warfarin and Vitamin-K), physiological parameters such as age, weight, height, clinical parameters such as diagnosed disorders, blood test results, chemistry, etc, a demographic parameter such as gender for birth-control pills, a physiological parameter such as blood pressure, heart rate and the like and a treatment related parameter (for example, receiving treatment for hyperkalemia when the offending drug is still active).

Each parameter occupies a specific element of the module and is identified by a specific location (numerical) or tag (code). In addition, as is mentioned above, each parameter is assigned a value which can be Boolean (e.g. true or false with respect to diagnosis, drug prescribed, past visit in a particular specialty clinic), discrete (e.g. number of times a particular drug has been prescribed in the past, age in months) or continuous (e.g. a clinical parameter such as blood count). The discrete or continuous value assigned to each element can be normalized to lie within a predefined range or have certain desirable statistical properties.

The system of the present invention can further include an inference engine for comparing, based on the identifiers, values of at least a portion of the elements of a module of a specific subject (patient) to a plurality of modules of diagnosed subjects in order to derive medically relevant information that is not represented in the module of the specific subject.

In such an approach, the module data of the specific subject is compared to data clustered from the plurality of subjects in the database. For example, using a clustering algorithm like K-means, the modules of a plurality of subjects in the database can be divided into K clusters. For a given subject in the database, incomplete data about that subject's medical status can then be probabilistically inferred by sampling the relevant property from other subjects belonging to the same cluster as the subject, for which this property is present in the records.

Alternatively, statistical characteristics of historical data of diagnosed subjects can be used to construct a model using supervised learning algorithms for classification or regression. The resulting model can then be applied to the module data of the specific subject in order to obtain a prediction of the class that subject belongs to (in the case of discrete classification, such as "has diagnosis X"), or a likely value for a given property (the response variable in the case of a regression model, such as "probability to develop diabetes in the next 5 years").

One preferred use of the present database and system is identifying drug prescription errors.

In such cases, the module data is compared to the clustered or modeled data in order to identify a probable prescription error based on frequency of prescription of the drug in diagnosed subjects. Such identification can be based on:

(i) determining a distance between clustered values and values of respective module elements of the specific subject
(ii) averaging the probability of drug D being given to subject S over all subjects in the DB (rather than limiting to those closest to S), with averaging weight inversely proportional to each subject's distance from S;
(iii) infer a probability for a discrete event, or a likely value for a continuous variable, from the statistics of the relevant property among all members of the cluster S belongs to;
(iv) train a classifier on the data, then apply it to S to get a prediction of its class; and/or
(v) train a regression model on the data, then apply it to S to get a likely value for the dependent variable of interest.

Approach (i) above is one presently preferred approach. When comparing a module element value to clustered data, a predetermined distance is determined by embedding the modules (of diagnosed subjects) in a vector space through a smooth mapping function and then measuring the distance between the mapped points in that space using a metric induced by a properly defined norm in the vector space.

Once the medically-relevant information is obtained from the comparison, it is communicated to a user (physician or technician) via a display device.

The results can then be qualified by the user and feedback can be provided to the system in order to improve accuracy. For example, in cases where the system flags a prescription as being erroneous, a physician prescribing the drug can disregard the flag and provide feedback that the prescription is correct. In such cases, the system can update the module of the patient and also update the model/clustered data ( to induce 'learning') with respect to the specific drug prescribed. It will be appreciated that provided enough input, the latter can increase the accuracy of the model or clustered data with respect to specific elements (parameters) of the module over time.

The module of the present invention is preferably arranged as a finite dimension vector having a preset length. The vector represents a time-related pattern of demographic data, prescriptions, diagnoses, hospitalizations, lab test results and/or medical procedures.

The system of the present invention can integrate with an EMR system to electronically obtain historical patient records as well as any new information related to the patient. The System can be used in a hospital setting, in a community settings, in a pharmacy setting, in an insurance company setting, in a pharmacy or pharmacy benefits manager (PBM) setting, or in a combination of the above.

The present system can evaluate, in real time, any prescription or medical order entered by a physician using the EMR system and provide real time feedback with respect to the prescription (e.g. flag probable prescription error) or order.

With respect to prescription errors, the present system can provide feedback (error alerts) with respect to two different types of events: prescriptions that were erroneous at the time of their entry; and prescriptions that were correct at the time of entry, but have become erroneous due to new laboratory results or diagnoses which have been entered into the EMR system and altered the patient's profile.

The present system is also particularly suitable for identifying several additional scenarios related to medication errors, including:

i. Medication mix-up - the wrong medication is prescribed to a patient.
ii. Patient mix-up - the wrong patient is prescribed the medication.
iii. Drug dosage outliers
iv. Drug vs. laboratory test incompatibilities - prescribe medications which may be hazardous due to laboratory test abnormalities (i.e. prescribe ACE inhibitors to a patient with hyperkalemia)
v. Drug vs. diagnosis incompatibilities - prescribe medications which may be hazardous due to a specific medical condition (i.e. provide anti-coagulants to a patient with recent intra-cranial bleeding)
vi. Other, less obviously defined, drug vs. "patient profile" incompatibilities - identify and warn about medications which are uncommonly used in the clinical setting of the patient (i.e. the use of vasopressors for a patient with no clinical justification according to vital signs, blood tests, chronic and acute diagnoses)

To identify such errors, the present system uses a multi-layered approach for analysis of a patient cohort data, employing the following procedures (described in detail below):

i. Calculate prescription statistics per generic drug at different ATC (Anatomical Therapeutic Chemical classification system) levels.
ii. Calculate prescription statistics per prescribing physician
iii. Calculate prescription statistics per unit (i.e. hospital department)
iv. Check a distinct set of rules for identifying drug-patient incompatibilities
v. Use clustering algorithms to cluster patient data (vector data) according to their clinical/laboratory/pharmaceutical data and evaluate if a prescription is an outlier
vi. Use machine learning algorithms to train and fine tune a drug compatibility model according to the prescribing physician, nurse or pharmacist response to the alert.

Although the present system is particularly useful in identifying medication related errors it can also be used to provide the following:

(i) probable diagnoses for a subject at a given point in time;
(ii) suggestions for a specific test to be performed;
(iii) suggestions for a specific drug to be prescribed;
(iv) suggestions for alternatives to subject's currently prescribed medications;
(v) probability of subject developing a specific condition within a given time frame; and/or
(vi) a likely stage of a condition in subjects having a gradually developing condition.

The following describes the invention in more detail starting with the patient module (vector).

*Module*

The module is constructed from a snapshot of a subject's health record at a given point in time. The health record data is processed and a formal representation of the data as a numerical vector of fixed length — $\boldsymbol{R} = (f1, f2, f3, ..., fN)$ is generated.

Each element of this vector is a "feature" of the patient's representation at the given point in time.

The vector generation process has 3 main stages: data extraction, data encoding, and feature calculation:

a. In the data extraction stage, textual data in the medical record is represented using predefined codes. For example, a diagnosis of "Subtrochanteric fracture of femur" can be transformed to its ICD10 code S72.2. Natural Language Processing (NLP) techniques can be used to identify words and phrases within free text stored in the EMR, from which diagnoses and other informative data elements can be thus extracted.

b. In the data encoding stage, the extracted data is transformed into purely numerical form. For example, in the previous example, the ICD10 code S72.2 can be transformed to an index 38172 using an enumeration table of all ICD10 codes.

c. In the feature calculation stage, predefined elements of the representation are calculated based on the numerical representation of the data extracted from the patient record. The final representation of the patient record is an ordered set of all the features calculated at this stage. This is best explained using some examples:

> i. Feature number 16 in the representation may contain the average number of hospitalizations the patient had in the year prior to the current prescription date. To calculate that feature, the System would go over the hospitalization table, and sum the number of entries whose dates are within the relevant range.
> ii. Features 152-3151 may contain indicator variables for 3000 major diagnoses at any time in history. Each of these would be 1 if the patient has ever been diagnosed with the respective diagnosis and 0 otherwise.
> iii. Features 3152-6151 may contain indicator variables for the occurrence of the same diagnoses during the last 3 months.
> iv. Feature 7329 may contain the total number of times the patient has been prescribed a drug classified as an NSAID (ATC code M01A) during the last month.

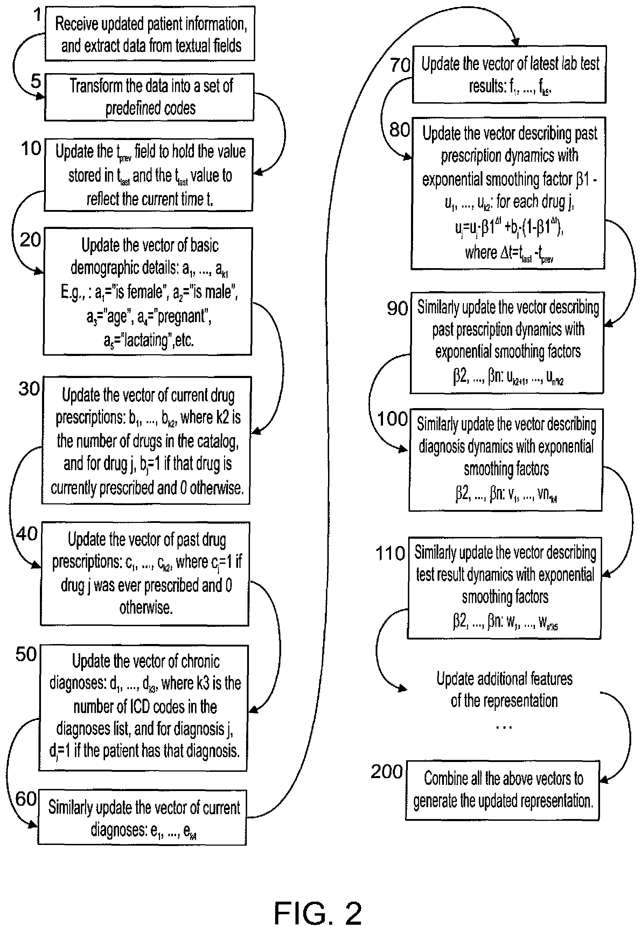

Figure 2 illustrates construction of such a vector in accordance with the teachings of the present invention.

At stage 1, updated patient information is received and data is extracted from textual fields. At stage 5, the data are transformed into predefined codes. In stage 10, the latest patient record modification time - $t_{last}$ - is updated to hold the current time, and the previous modification time - $t_{prev}$ - is recorded. At stage 20, features corresponding to basic demographic data are updated. For example, if the patient has become pregnant since the previous record update, field 5, corresponding to "is pregnant" would be changed from value 0 to 1. At stage 30, current drug prescriptions are updated. For example, if the patient has come off drug A and was prescribed drug B, the field corresponding to "active prescription of drug A" would be assigned value 0, and the one corresponding to "active prescription of drug B" would be assigned value 1. At stage 40, past drug prescriptions are updated - the fields corresponding to all currently active prescriptions, within the vector of past prescriptions, are assigned value 1. At stage 50, the vector of chronic diagnoses is updated. For example, if the patient was diagnosed with ICD9 code 250.00 ("diabetes mellitus type II, without complications"), the field corresponding to that diagnosis code would be assigned value 1. At stage 60, the vector of current diagnoses (chronic or acute) is similarly updated. At stage 70, the vector of latest lab test results is similarly updated. At stage 80, a vector describing past prescription dynamics is updated. In an example previously given, the average number of prescriptions is used to demonstrate how a certain aspect of prescription dynamics can be recorded. Here a slightly different approach is demonstrated, using exponential moving averages (EMAs) to achieve a similar goal. To this end, a number $\beta1$ is fixed between 0 and 1. For each drug i in the drug catalog, a number $u_i$ representing the EMA of drug i with smoothing factor $\beta1$ is kept and updated. At each update, the time difference ($\Delta t$) is calculated from the last update as follows: $\Delta t = t_{last} - t_{prev}$. The number $u_i$ is then multiplied by factor $\beta1^{\Delta t}$. If drug i is currently prescribed, $u_i$ is further incremented by $(1 - \beta1^{\Delta t})$. The exponential smoothing factor $\beta1$ controls the "memory length" of the feature $u_i$ - smaller values of $\beta1$ would result in "less memory", resulting in a number which is affected mainly by recent prescription changes. Larger values would result in a number which is also affected by changes occurring in the more distant past. By using several different smoothing factors, complex dynamic patterns can be recorded using a small number of features. Hence, at stage 90, a similar process is repeated using additional smoothing factors $\beta2, ..., \beta n$. At stage 100, a similar process is used to update diagnosis dynamics with multiple smoothing factors $\beta1, ..., \beta n$. At stage 110, a similar process is used to update lab result dynamics with multiple smoothing factors $\beta1, ..., \beta n$. Similar means can be used to update additional feature vectors until finally, at stage 200, all of the updated vectors, each having a predefined length, are combined at a predefined order to obtain a single vector of predefined length holding the updated representation.

*Database*

The database component of the present system includes data from the EMR (historical and current) which is needed for analysis, as well as binary code of the representation modules and class objects serialization as detailed herein. The database is relational and accessed via standard SQL (an NoSQL database or a flat file storage database can also be used).

The following describes one possible embodiment of the database and its subsets. The database subsets can include additional tables or additional data fields can be added to any of the existing tables.

Patient database

> i. Patient information, including demographics
> ii. Diagnosis, including all diagnoses, chronic or acute, obtained from the family physician or during hospital admission/discharge, as well as surgical procedures, coded by ICD-9 and/or ICD-10 codes

**iii.** Blood tests, including complete blood count, chemistry and electrolytes, hormonal/vitamin levels, coagulation factors, cultures and blood gasses

**iv.** Parameters obtained by nursing staff, including heart rate, blood pressure, saturation, weight, glucose etc.

**v.** Hospitalization and outpatient clinic data, including admission/release dates, urgent/planned, hospital and unit/clinic

**vi.** Chronic medication data, including details of chronic medications taken by patients prior to hospital admission

**vii.** Historical prescriptions data, including drug, dose, rout of administration, start/end date of prescription, Drug Daily Dose (DDD), prescribing physician etc.

**viii.** Alert response details, including details of the physician response to each of the alerts the System generated.

Administrative database

Includes tables which are used for analysis but are not patient-specific.

**i.** Drug list, including available drugs in the medical facility, their ATC coding, generic form, administration rout etc.
**ii.** ICD-9 and ICD-10 coding hierarchy
**iii.** Institutional units' details (i.e. departments, outpatient clinics etc).

Patient object database

Modules containing the mathematical representation of the patients records (in vector format) will be stored as blobs (binary large objects). The patient object database contains serialized representations of patient 'objects'. When the data pertaining to a specific patient needs to be online, the corresponding serialized object will be loaded into memory, and will be available for update and analysis of prescriptions. In a hospital setting, the object will be loaded when the patient registers at the ER, is admitted or arrives at the outpatient clinic. In a community setting, the object will be loaded when the patient is scheduled to visit the family physician, nurse or consultant. When new data regarding the patient is received (i.e. new blood tests) the patient object will be loaded, updated, prescription errors will be generated (if needed) and the object will be subsequently serialized and saved back in the database.

Statistical database

The statistical database contains statistical data, including prescription statistics per physician, unit or hospital, associations between drug pairs, associations between diagnosis pairs, associations between drugs and diagnoses, and blood test value distribution per medication and per diagnosis. It also includes prescription distribution, including details of each medication distribution of a specific hospital prescription and an indication whether the patient actually received the drug (i.e. if a prescription was given for IV. Ceftriaxone 1g X1/D for one week, a record will be constructed for each of the 7 times the medication should be distributed and an indication will be made for each one if the patient actually received the drug by the nurse at that time).

***Rule-based Inference Engine***

The rule-based inference engine applies a set of inference rules to the combination of patient profile and prescription data, to identify certain classes of high-risk mistakes. The general form of an inference rule is

*IF (X(patient) AND Y(drug)) THEN issue alert S*

In this formulation, *X* and *Y* are Boolean functions on the space of patient profiles and generic drug names, respectively, whereas S is some alert string.

This can be exemplified by the following simple rule specification: X(patient) ≡ {1 if patient's blood sugar level ≤ 70 mg/dL; 0 otherwise}; Y(drug) ≡ {1 if drug is insulin; 0 otherwise} ∎ ; S ≡ "Giving insulin to a patient with hypoglycemia"

Clearly, the very simple example above can be generalized in order to cover patient conditions which are defined in a much more complex manner. In particular, the Boolean function describing the patient condition does not necessarily have to be defined by a human expert. Instead, it can, for example, signify that the patient was assigned a certain label by another algorithm. This can be useful in combination with, e.g., clustering algorithms that can implicitly identify certain hard-to-define conditions even in the absence of an explicit diagnosis in the patient's health record (detailed description provided below under "Machine Learning Inference Engine").

Following is a list of some useful rules that can be used to identify common life-threatening errors:

Drug - lab tests incompatibility rules

    i. Anti diabetic drug to a patient with hypoglycemia
    ii. Potassium/ACE-I/ARB/potassium sparing diuretics to a patient with hyperkalemia
    iii. Hypertonic saline to a patient without hyponatremia
    iv. NSAIDs to a patient with renal failure
    v. Anti thrombotics to a patient with thrombocytopenia
    vi. Opiates to a patient with hypercarbia
    vii. HGM-CoA reductase inhibitors/Amiodarone to a patient with elevated liver enzymes
    viii. Anti coagulants to a patient with highly elevated INR

Drug — diagnosis incompatibility rules

Drug - diagnosis incompatibilities may be inferred when an explicit diagnosis is documented in the patient's health record. However, as mentioned above, such incompatibility may be inferred even in the absence of an explicit diagnosis, relying instead on an algorithm for producing a probabilistic diagnosis. For example, the Machine Learning Inference Engine of the present system can tag a patient as having a high probability for being diagnosed with diabetes given high blood glucose levels, or recorded visits to a diabetes clinic.

Examples of drug-diagnosis incompatibility rules are:

    i. Anti diabetic drug to a patient without diabetes
    ii. Anti neoplastic to a patient with no malignancy
    iii. Anti coagulation with no indication
    iv. Anti thrombotics to a patient with high risk of bleeding

### *Statistical Inference Engine*

The Statistical Inference Engine infers likely drug incompatibilities from basic statistical properties. Examples of such inferences include:

    i. Rarely prescribed drugs - a prescription for a drug which is very rarely prescribed is likely to result from an error. This simplest statistical inference rule can be customized to specific scenarios, measuring prescription frequency within a given organization, in a specific ward, or by a specific physician.
    ii. Specificity-based compatibility scoring - calculate for each drug family (ATC level 4) and diagnosis family (ICD level 3) the following:

        **Pdrug** the frequency of prescriptions involving drugs from this family;
        **Pdiagnosis** the frequency of patients with this diagnosis family; and
        **Pdiagnosis+drug** the frequency of a patient having this diagnosis and drug combination.

For each drug-diagnosis combination, calculate the specificity score:

$$S(drug,\ diagnosis) = log(pdiagnosis{+}drug\ /\ (pdrug \cdot pdiagnosis))$$

Given a specific prescription for DRUG and the set of the patient's diagnoses, issue an alert if (i) *S(DRUG, diagnosis)* < 1 for all of the patient's diagnoses; AND (ii) there is at least one diagnosis family DIAG in the diagnoses database for which *S(DRUG, DIAG)* ≥ 1.

### *Machine Learning Inference Engine*

The Machine Learning (ML) Inference Engine uses data-driven classification and regression techniques in order to identify cases where a prescription is likely to be erroneous. Some basic principles are common to the different algorithms that are used within the ML Engine in the current invention, and to other algorithms that can be used in subsequent implementations of the present invention:

1. Historical data from the health records of many patients is used as a *training set* for the algorithms, serving as the basis on which the predictive model is tuned and appropriate model parameter values are obtained.

2. *Unsupervised Learning* techniques are used to identify patterns within the historical data corpus, supporting the identification of future prescriptions as conforming to the normal pattern of being *outliers.*

3. The feedback of physicians, nurses, and other users of the System to the alerts presented to them forms *labeled examples* for *Supervised Learning* algorithms, allowing the System to more accurately tune its predictive algorithms and in particular to adjust the sensitivity threshold of its alerts to specific users.

The following exemplifies each of the above principles:

Metric analysis for outlier detection

The numerical representation of a patient's health record can be regarded as a point in a multi-dimensional vector space (with the dimension being equal to the number of features in the representation). Given a *metric* (a "distance function") in this space, outlier detection within this metric space can point out potentially erroneous prescriptions. Given a patient with representation vector $R$, who is receiving prescription $p,$ this approach is based on measuring the frequency of the prescription $p$ among patients whose representation is close to $R.$

Such an approach can be effected as follows:

i. Fix some natural number $N$ (e.g. 50), and a threshold $\theta$ ($0 < \theta < 1$). The values of these parameters can be optimized based on local search in parameter space. Find the $N$ patients whose representations are closest to $R$ among those who have received prescription $p,$ and calculate the average distance $d$ between $R$ and the representations of these patients. Also calculate the average distance $D$ between $R$ and its $N$ nearest neighbors. Issue an alert if $d > D \cdot \theta.$

ii. Fix some natural number $N$ (e.g. 1000), and two thresholds $\theta1$ and $\theta2$. Calculate the frequency $f$ of prescription $p$ among the $N$ patients whose representations are closest to $R$. Also calculate the mean $\mu$ and standard deviation $\sigma$ of the frequency of prescription $p$ within random draws of $N$ patients from the population. Issue an alert if (($\sigma < \theta1$) AND ($f - \mu > \sigma \cdot \theta2$)).

Using Clustering algorithms for outliers' detection

One can apply clustering algorithms such as KNN ("K Nearest Neighbors") or K-Means in order to classify all the patients into a relatively small number of clusters. Intuitively, these clusters would represent groups of patients with similar conditions. For example, most patients with type-2 diabetes are likely to form a cluster due to proximity in the values of features related to blood-glucose test results, diabetes clinic visits, BMI, etc.

Once clustering has been performed, the cluster IDs of a patient can be used guide outlier detection. If, for example, a given drug is rarely prescribed in all of the clusters a patient participates in, then its prescription to that patient is more likely to be an error.

Alert fine-tuning using supervised learning techniques

Historical data about prescriptions in general does not contain "labeling" of whether a particular prescription was adequate or erroneous, and is thus ill- suited for application of *supervised learning* algorithms.

The situation is different with respect to the smaller set of prescriptions for which alerts have been issued. For these prescriptions, Physician/Nurse responses to the alert can indeed serve as reliable indicators for prescription adequacy. The present system employs a second algorithmic layer of supervised learning, which utilizes user feedback to fine- tune the alerts and personalize them.

In the following example, a "labeled sample" is a triplet $<R,p,l>,$ where $R$ is a patient representation at some point in time; $p$ is a prescription given to the patient at that time, which resulted in an alert by the present system; and $l$ is a binary label: 0 if the alert was rejected and 1 if it was accepted.

By training a classification algorithm on the set of labeled samples, one can improve the accuracy of the underlying alerts, identifying prescriptions which are misclassified by the rule-based, statistical, or outlier-detection methods and should be suppressed. For example, the labeled data set can be used to train a Support Vector Machine (SVM) to discriminate between true and force alert. By splitting the labeled data into a training set and a test set, the performance of the SVM can be cross-validated, and its parameters can be adjusted in order to reach an appropriate precision-recall tradeoff. Such tuning is important in order to balance the desire to identify as many errors as possible with the need to reduce the false alarm rate in order to avoid the phenomenon known as 'alert fatigue' among users who are exposed to too many false alarms.

In addition to improving the overall performance of the system, the supervised learning stage can also be used to personalize the alerts to usage patterns of different users. A "context variable" C can be added to the labeled sample: *<R,p,C,l>* to identify the prescribing physician, the prescribing physician's specialty, the ward or clinic type if appropriate, etc. By taking the context into account in the classification process, the classification algorithm can make different decisions based not only on the patient condition but also on the context.

For example, consider an oncologist who regularly prescribes chemotherapy. In some cases, the chemotherapy prescription can occur when there are still few indications of malignancy, if any, in the patient's medical record. For another physician, such a prescription would likely result in an alert, but in the case of the oncologist it would not be regarded as exceptional, unless some other factors give such indication (e.g., it's a drug never before prescribed by this physician). Similarly, a context variable may represent different priorities and guidelines that have been set by specific wards and organizations. It is easy to see how other classification algorithms, such as, e.g., Decision Trees or Linear Discriminant Analysis can be used in the above instead of SVM.

### I/O Interfaces

The present system can be based on a web server technology. In such an embodiment, the interface with EMR and CPOE systems is based on messages being sent back and forth between these three systems, issuing and responding to data queries. For the sake of simplicity, the following example does not make a distinction between the CPOE system and the EMR system, and considers the former as a component within the latter.

The present system is kept updated with regards to details of patients whose prescriptions need analysis by querying the EMR system or receiving 'push' updates from it once a field is updated. Examples of data used by the System include:

i. Patient demographics
ii. Hospitalization records
iii. Outpatient clinics records
iv. Diagnosis (past, chronic and acute)
v. Blood tests history
vi. Chronic medications
vii. Medication prescription history
viii. Vital signs history

The messages sent from the EMR to the present system contain medication prescriptions for analysis, patient specific data, administrative data as well as indicators of events, such as, for example, the registration of a patient in the emergency room, hospital department or outpatient clinic.

Events sent from the EMR to the present system are used to signal that specific data will be needed soon and therefore the present system is required to obtain/load it. Examples of such events are:

i. Incoming patient (hospital admission, outpatient clinic etc.) - need to load and update patient details
ii. Outgoing patient (hospital discharge, left the outpatient clinic etc.) - need to save and unload patient data objects
iii. User entrance to prescription subsystem in EMR - need to update all patient specific data for analysis of incoming prescriptions
iv. Request for prescription error and respond report - need to create a report containing all identified errors within a time range and the user response to these errors (i.e. accept or decline)
v. Incoming blood test or other laboratory results - contains all relevant blood test results just received by the EMR and sent to the System for re-analysis of active prescriptions

Messages sent from the System to the EMR contain requests for patient/administrative data and medication error alerts. Data requests from the System to the EMR include requests which are a response to an incoming event (for example: "get patient details" soon after receiving an event which indicated that a patient was admitted to the hospital), or requests with a chronological basis (for example: "get vital signs", by which the System updates itself on up-to-date vital signs obtained for all "active" patients).

Examples of data requests include:

i. Get patient details - for a list of patients for a specific time range
ii. Get vital signs - for a list of patients for a specific time range
iii. Get in/out-patient visits - for a specific patient for a specific time range
iv. Get blood tests - for a list of patients for a specific time range
v. Get diagnosis - for a list of patients

vi. Get drug allergies - for a list of patients
vii. Get chronic/active medications - for a list of patients
viii. Get institution drug list
ix. Get institution department/unit/outpatient list
x. Get institution user list (physician/nurses and pharmacists)

Alerts may be issued at the time of the prescription, and/or at a later stage, when new blood tests/diagnosis are noted and the medication is still active (mainly, drug-blood test incompatibilities and drug-diagnosis incompatibilities).

The communication flow between the System and the EMR with regards to prescription analysis and alert can be as follows:

i. From EMR to System: prescription details
ii. From System to EMR: alert message (if appropriate)
iii. From EMR to System: user response to alert (accept or decline)

Additional objects, advantages, and novel features of the present invention will become apparent to one ordinarily skilled in the art upon examination of the following examples, which are not intended to be limiting.

## EXAMPLES

Reference is now made to the following example, which together with the above descriptions, illustrate the invention in a non limiting fashion.

### *Identifying a drug prescription error using a data-driven approach*

A simplified example with a single drug family and a two-dimensional patient representation is used to illustrate the present data-driven approach for identifying probable prescription errors. The drug family in question is statins (ATC code C10AA), and the two features chosen to describe patients are their age, and their latest GPT liver enzyme test result.

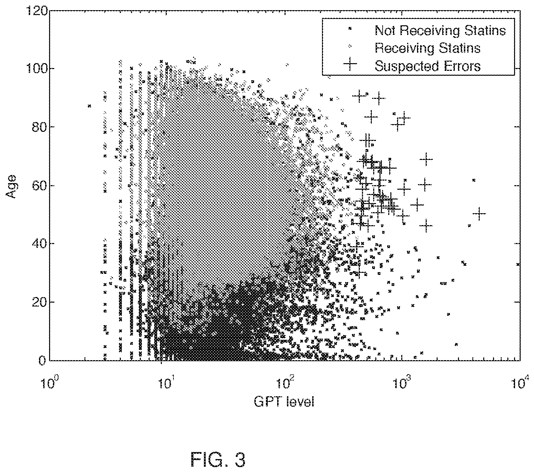

Figure 3 illustrates distributions of patients receiving statins (black dots) and not receiving statins (gray dots) along the two dimensions of the chosen representation. Points corresponding to the representations of 100,000 patients from each of the two groups are plotted. The cross-shaped points represent patients who were prescribed statins, for whom the prescription was identified as a likely error.

The algorithm of the present invention was used to identify these probable errors as follows:

a. Normalize the scales of the two dimensions, dividing each by the standard deviation of the sample along that dimension;
b. For each point p representing a patient who was prescribed statins, find the k nearest points q1, q2, ..., qk corresponding to other patients prescribed with statins, with distance measured in the metric induced by the $L\infty$ norm, i.e., the maximum distance along all coordinates;
c. Calculate the k distances d1, d2, ..., dk between p and each of p1, ..., pk;
d. Calculate the average, Dp, of (dl, ... dk);
e. Calculate the mean, M, and the standard deviation, s, of Dp, for all points p representing patients prescribes with statins; and
f. Identify as a potential prescription error any patient for whom D is in the top 1/2000 of the range of D values.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

### *References*

1. Velo, G.P. and P. Minuz, Medication errors: prescribing faults and prescription errors. Br J Clin Pharmacol, 2009. 67(6): p. 624-8.
2. How to reduce prescribing errors. Lancet, 2009. 374(9706): p. 1945.
3. Gandhi, T.K., et al., Outpatient prescribing errors and the impact of computerized prescribing. J Gen Intern Med, 2005. 20(9): p. 837-41.

4. Preventing Medication Errors: A $21 Billion Opportunity. National Priorities Partnership and National Quality Forum, 2010.

5. James, J.T., A new, evidence-based estimate of patient harms associated with hospital care. J Patient Saf, 2013. 9(3): p. 122-8.

6. Devine, E.B., et al., The impact of computerized provider order entry on medication errors in a multispecialty group practice. J Am Med Inform Assoc, 2010. 17(1): p. 78-84.

7. Jani, Y.H., et al., Electronic prescribing reduced prescribing errors in a pediatric renal outpatient clinic. J Pediatr, 2008. 152(2): p. 214-8.

8. Kaushal, R., et al., Electronic prescribing improves medication safety in community-based office practices. J Gen Intern Med, 2010. 25(6): p. 530-6.

9. Classen, D.C. and D.W. Bates, Finding the meaning in meaningful use. N Engl J Med, 2011. 365(9): p. 855-8.

10. Aronson, J.K., Medication errors: what they are, how they happen, and how to avoid them. Qjm, 2009. 102(8): p. 513-21.

## Claims

1. A medical data system (10) comprising:
a data unit (12) for storing modules (14) representing medical records of subjects, the medical data system being **characterized in that** :

   each module is represented as a vector including a
   plurality of vector elements (20) each representing a dimension of said vector and a medically-relevant parameter of a subject, wherein each vector element is assigned a specific identifier (22) in said vector and a numerical value (24) corresponding to said medically-relevant parameter; and **in that**:
   further comprises an inference engine for comparing, based on identifiers of vector elements, values of at least a portion of said elements of a module of said subject to at least one model constructed from statistical characteristics of historical data of diagnosed subjects to thereby identify a drug prescription-related parameter not present in a medical file of said subject.

2. The medical data system of claim 1, wherein said medically-relevant parameter is selected from the group consisting of a demographic parameter, a physiological parameter, a drug prescription-related parameter, a disease related parameter, and a treatment related parameter.

3. The medical data system of claim 1, wherein said drug prescription-related parameter is a probable drug prescription error.

4. The medical data system of claim 3, wherein said probable prescription error is based on binary classification of said at least one model.

5. The medical data system of claim 3, wherein said probable prescription error is based on continuous regression against said at least one model.

6. The medical data system of claim 1, wherein said vector has a preset length.

7. The medical data system of claim 1, wherein said vector represents a time-related pattern of demographic data, prescriptions, diagnoses, hospitalizations, lab test results and/or medical procedures.

## Patentansprüche

1. Medizinisches Datensystem (10), aufweisend:
eine Dateneinheit (12) zum Speichern von Modulen (14), die medizinische Aufzeichnungen von Subjekten darstellen, wobei das medizinische Datensystem **dadurch gekennzeichnet ist, dass**:

   jedes Modul als ein Vektor dargestellt wird, der eine Vielzahl von Vektorelementen (20) enthält, von denen jedes eine Dimension des besagten Vektors darstellt, sowie einen medizinisch relevanten Parameter eines Subjekts, wobei jedem Vektorelement eine spezifische Kennung (22) in dem Vektor und ein numerischer Wert (24) entsprechend dem medizinisch relevanten Parameter zugewiesen ist; und dadurch, dass: es weiterhin eine Inferenzmaschine zum Vergleichen von Werten mindestens eines Teils der besagten Elemente eines Moduls des Subjekts mit mindestens einem Modell auf der Grundlage von Kennungen der Vektorelemente aufweist, wobei das Modell aus statistischen Merkmalen von historischen Daten diagnostizierter Subjekten gebildet ist, um dadurch einen arzneimittelverschreibungsbezogenen Parameter zu identifizieren, der in einer medizinischen Akte des Subjekts nicht vorhanden ist.

**2.** Medizinisches Datensystem nach Anspruch 1, wobei der besagte medizinisch relevante Parameter aus der Gruppe ausgewählt ist, die aus einem demographischen Parameter, einem physiologischen Parameter, einem arzneimittelverschreibungsbezogenen Parameter, einem krankheitsbezogenen Parameter und einem behandlungsbezogenen Parameter besteht.

**3.** Medizinisches Datensystem nach Anspruch 1, wobei der besagte arzneimittelverschreibungsbezogene Parameter ein wahrscheinlicher Medikamentenverschreibungsfehler ist.

**4.** Medizinisches Datensystem nach Anspruch 3, wobei der besagte wahrscheinliche Verschreibungsfehler auf einer binären Klassifizierung des besagten mindestens einen Modells basiert.

**5.** Medizinisches Datensystem nach Anspruch 3, wobei der besagte wahrscheinliche Verschreibungsfehler auf einer kontinuierlichen Regression gegen das besagte mindestens eine Modell basiert.

**6.** Medizinisches Datensystem nach Anspruch 1, wobei der besagte Vektor eine voreingestellte Länge hat.

**7.** Medizinisches Datensystem nach Anspruch 1, wobei der besagte Vektor ein zeitbezogenes Muster von demografischen Daten, Verschreibungen, Diagnosen, Krankenhausaufenthalten, Labortestergebnissen und/oder medizinischen Verfahren darstellt.

## Revendications

**1.** Système de données médicales (10) comprenant :

une unité de données (12) pour stocker des modules (14) représentant des dossiers médicaux de sujets, le système de données médicales étant **caractérisé en ce que** :

chaque module est représenté comme un vecteur incluant une pluralité d'éléments vectoriels (20) représentant chacun une dimension dudit vecteur et un paramètre médicalement approprié d'un sujet, dans lequel chaque élément vectoriel se voit attribuer un identificateur spécifique (22) dans ledit vecteur et une valeur numérique (24) correspondant audit paramètre médicalement approprié ;
et **en ce que** :
il comprend en outre un moteur d'inférence pour comparer, sur la base d'identificateurs d'éléments vectoriels, des valeurs d'au moins une portion desdits éléments d'un module dudit sujet à au moins un modèle construit à partir de caractéristiques statistiques de données historiques de sujets diagnostiqués pour identifier de ce fait un paramètre lié à une prescription de médicament non présent dans un fichier médical dudit sujet.

**2.** Système de données médicales selon la revendication 1, dans lequel ledit paramètre médicalement approprié est sélectionné dans le groupe constitué par un paramètre démographique, un paramètre physiologique, un paramètre lié à une prescription de médicament, un paramètre lié à une maladie et un paramètre lié à un traitement.

**3.** Système de données médicales selon la revendication 1, dans lequel ledit paramètre lié à une prescription de médicament est une erreur probable de prescription de médicament.

**4.** Système de données médicales selon la revendication 3, dans lequel ladite erreur probable de prescription est basée sur une classification binaire dudit au moins un modèle.

**5.** Système de données médicales selon la revendication 3, dans lequel ladite erreur probable de prescription est basée sur une régression continue contre ledit au moins un modèle.

**6.** Système de données médicales selon la revendication 1, dans lequel ledit vecteur a une longueur définie à l'avance.

**7.** Système de données médicales selon la revendication 1, dans lequel ledit vecteur représente un schéma temporel de données démographiques, de prescriptions, de diagnostics, d'hospitalisations, de résultats de tests de laboratoire et/ou de procédures médicales.

FIG. 1

**1** Receive updated patient information, and extract data from textual fields

**5** Transform the data into a set of predefined codes

**10** Update the $t_{prev}$ field to hold the value stored in $t_{last}$ and the $t_{last}$ value to reflect the current time t.

**20** Update the vector of basic demographic details: $a_1, ..., a_{k1}$ E.g., : $a_1$="is female", $a_2$="is male", $a_3$="age", $a_4$="pregnant", $a_5$="lactating",etc.

**30** Update the vector of current drug prescriptions: $b_1, ..., b_{k2}$, where k2 is the number of drugs in the catalog, and for drug j, $b_j$=1 if that drug is currently prescribed and 0 otherwise.

**40** Update the vector of past drug prescriptions: $c_1, ..., c_{k2}$, where $c_j$=1 if drug j was ever prescribed and 0 otherwise.

**50** Update the vector of chronic diagnoses: $d_1, ..., d_{k3}$, where k3 is the number of ICD codes in the diagnoses list, and for diagnosis j, $d_j$=1 if the patient has that diagnosis.

**60** Similarly update the vector of current diagnoses: $e_1, ..., e_M$

**70** Update the vector of latest lab test results: $f_1, ..., f_{k5}$.

**80** Update the vector describing past prescription dynamics with exponential smoothing factor β1 - $u_1, ..., u_{k2}$: for each drug j, $u_j = u_j \cdot \beta 1^{\Delta t} + b_j \cdot (1 - \beta 1^{\Delta t})$, where $\Delta t = t_{last} - t_{prev}$

**90** Similarly update the vector describing past prescription dynamics with exponential smoothing factors β2, ..., βn: $u_{k2+1}, ..., u_{n \cdot k2}$

**100** Similarly update the vector describing diagnosis dynamics with exponential smoothing factors β2, ..., βn: $v_1, ..., vn_M$

**110** Similarly update the vector describing test result dynamics with exponential smoothing factors β2, ..., βn: $w_1, ..., w_{n \cdot k5}$

Update additional features of the representation

. . .

**200** Combine all the above vectors to generate the updated representation.

FIG. 2

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007219824 A1
- US 2009216555 A1

**Non-patent literature cited in the description**

- **VELO, G.P. ; P. MINUZ.** Medication errors: prescribing faults and prescription errors. *Br J Clin Pharmacol,* 2009, vol. 67 (6), 624-8
- How to reduce prescribing errors. *Lancet,* 2009, vol. 374 (9706), 1945
- **GANDHI, T.K. et al.** Outpatient prescribing errors and the impact of computerized prescribing. *J Gen Intern Med,* 2005, vol. 20 (9), 837-41
- Preventing Medication Errors: A $21 Billion Opportunity. *National Priorities Partnership and National Quality Forum,* 2010
- **JAMES, J.T.** A new, evidence-based estimate of patient harms associated with hospital care. *J Patient Saf,* 2013, vol. 9 (3), 122-8
- **DEVINE, E.B. et al.** The impact of computerized provider order entry on medication errors in a multispecialty group practice. *J Am Med Inform Assoc,* 2010, vol. 17 (1), 78-84
- **JANI, Y.H. et al.** Electronic prescribing reduced prescribing errors in a pediatric renal outpatient clinic. *J Pediatr,* 2008, vol. 152 (2), 214-8
- **KAUSHAL, R. et al.** Electronic prescribing improves medication safety in community-based office practices. *J Gen Intern Med,* 2010, vol. 25 (6), 530-6
- **CLASSEN, D.C. ; D.W. BATES.** Finding the meaning in meaningful use. *N Engl J Med,* 2011, vol. 365 (9), 855-8
- **ARONSON, J.K.** Medication errors: what they are, how they happen, and how to avoid them. *Qjm,* 2009, vol. 102 (8), 513-21